(19) Europäisches Patentamt
European Patent Office
Office européen des brevets

(11) Publication number: **0 366 205 B1**

(12) **EUROPEAN PATENT SPECIFICATION**

(45) Date of publication of patent specification: **26.01.94**

(51) Int. Cl.[5]: **C07D 303/16**, C07C 323/52, //C08G59/02

(21) Application number: **89202689.9**

(22) Date of filing: **24.10.89**

(54) **Novel polyglycidyl esters, their preparation and their use in thermosetting compositions.**

(30) Priority: **26.10.88 GB 8825057**

(43) Date of publication of application:
**02.05.90 Bulletin 90/18**

(45) Publication of the grant of the patent:
**26.01.94 Bulletin 94/04**

(84) Designated Contracting States:
**DE FR GB**

(56) References cited:
**DE-C- 1 904 110**
**DE-C- 1 942 836**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Smaardijk, Abraham Adriaan**
**Badhuisweg 3**
**NL-1031 CM Amsterdam(NL)**
Inventor: **Fennis, Paulus Jacobus**
**Badhuisweg 3**
**NL-1031 CM Amsterdam(NL)**
Inventor: **Van Reijendam, Jan Willem**
**Badhuisweg 3**
**NL-1031 CM Amsterdam(NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

This invention relates to novel polyglycidyl esters, a process for their preparation and their use in thermosetting compositions, particularly for use in solventless coatings and composites.

Diglycidyl esters of linear dicarboxylic acids such as adipic and sebacic acid are known e.g. from US Patent 3,053,855. Although such polyglycidyl esters and the products based thereon have been found to have very good outdoordurability performance properties, they suffer from a major deficiency, i.e. insufficient hydrolytic stability. Diglycidyl esters having improved hydrolytic stability have been described in German Published Patent Application 1942836, and are based on branched dicarboxylic acids having a linear $C_1$-$C_{10}$ alkylene chain carrying 2-3 $C_1$-$C_4$ alkyl substituents, special attention being focussed on the diglycidyl esters of 2,2,4- and 2,4,4- trimethyladipic acid. The improved hydrolytic stability of these diglycidyl esters being no doubt related to the presence of an $\alpha$-carbon atom, with respect to the carbonyl group, carrying one or two lower alkyl substituents. In this context it may be expected that polyglycidyl esters based on polycarboxylic acids wherein each of the $\alpha$-carbon atoms is di-substituted with a lower alkyl group, e.g. a methyl group, should be of great interest.

As a result of continuing and extensive research and experimentation, there was surprisingly found that it is possible to prepare such polyglycidyl esters, based on polycarboxylic acids carrying two alkyl substituents on each of the $\alpha$-carbon atoms. These polyglycidyl esters are novel compounds.

The invention provides therefore polyglycidyl esters of general formula

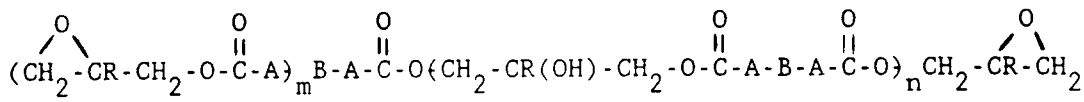 I

wherein R is H or $CH_3$; A represents an alkylene group $-CR^1_2-CH_2-$wherein each $R^1$ represents a $C_1$-$C_4$ alkyl group which may be the same or different, which group A is attached to the adjacent group B via the $-CH_2-$ group; n has a value in the range of from 0-10; m is 1 or 2; for m is 1 B represents a group $-SO_p-$ or a group $-SO_p-Q-SO_p-$ wherein p is 0, 1 or 2, and Q is a linear or branched alkylene group having from 2-10 carbon atoms, for m is 2 B represents a group $(SO_p-CH_2)_2-CH-SO_p-$, wherein p has the same meaning as hereinbefore.

In the context of the present invention the term polyglycidyl ester refers to glycidyl esters having on average more than one glycidyl ester group per molecule.

Depending on the type of acid from which they have been derived, as well as on their molecular weight, the polyglycidyl esters of the present invention may vary from low-viscosity, oil-type liquids to highly viscous or semi-solid products. The low-viscosity polyglycidyl esters may suitably be used as reactive diluent in thermosetting and chemically crosslinkable systems. The higher-viscosity products may be used e.g. as a binder component in coating compositions and/or composites. A further potential outlet envisaged for these glycidyl esters is as stabilizer e.g. in polymers. Except for a difference in viscosity and physical state, the polyglycidyl esters of general formula I will include products which may vary strongly in other characteristics such as glass-transition temperature (Tg) and hydrolytic stability. For applications which have a certain minimum requirement regarding the Tg a preferred class of such polyglycidyl esters are those wherein B is $-SO_p-Q-SO_p-$ and Q is an ethylene group, or wherein B is $-SO_p-$. Especially preferred are such polyglycidyl esters wherein p is 1 or 2 and more preferably p is 2. A class of polyglycidyl esters of general formula I of which the molecular structure may provide a positive contribution towards the Tg of said polyglycidyl esters or the products derived therefrom, are those wherein each $R^1$ is $-CH_3$. For applications wherein the requirements regarding hydrolytic stability are very strict, preferred polyglycidyl esters are those wherein p is O.

Although in theory the number of glycidyl groups per molecule of polyglycidyl ester will be equivalent to the functionality of the polycarboxylic acid on which they are based, in practice and depending the method of preparation, this number may be somewhat lower, the difference being made up by e.g. $\alpha$-glycol groups, i.e. hydrolysed glycidyl groups, chlorohydrin groups and carboxyl groups.

The preparation of the polyglycidyl esters of the present invention may conveniently be conducted by known processes for the preparation of polyglycidyl esters e.g. such as those which have been described in the hereinbefore mentioned German Patent Application, i.e.:

a) the reaction of the alkali metal salt of a suitable polycarboxylic acid with an excess of an epihalohydrin or a $\beta$-methylepihalohydrin and subsequently separating off the co-formed alkali metal halogenide,

$b_1$) the reaction of a polycarboxylic acid with an excess of an epihalohydrin or a $\beta$-methylepihalohydrin, generally >2 mol of epihalohydrin per carboxyl group, in the presence of a suitable catalyst such as a tertiary amine, a quaternary ammonium salt or an ion-exchange resin, whereby the intermediately formed halohydrin group is converted to an epoxy group under the production of the corresponding glycerindihalogenhydrin as a result of the reaction between the halohydrin formed and the excess of epi-halohydrin or $\beta$-methylepihalohydrin, and

$b_2$) a reaction between a polycarboxylic acid and epihalohydrin or $\beta$-methylepihalohydrin in the presence of a tertiary amine or a quaternary ammonium salt and employing a dehydrohalogenation-agent such as water-free sodium hydroxide or related inorganic compounds.

Preferably the epihalohydrin and/or the $\beta$-methylepihalohydrin which may be used in the preparation of the polyglycidyl esters of the present invention are epichlorohydrins, epichlorohydrin being especially preferred.

It will be understood by those skilled in the art that the value for n will be largely determined by the nature of the process used for the preparation of the polyglycidyl esters as well as by the excess of epihalohydrin compound employed. Generally the polyglycidyl esters will be a mixture of such esters having different values for n. If desirable it is also possible to prepare such polyglycidyl esters by reaction of a polycarboxylic acid of general formula II, as given hereinafter, with a molar excess of a polyglycidyl ester as hereinbefore described e.g. a polyglycidyl ester of general formula I for which n = o, in the presence of a suitable catalyst.

The polycarboxylic acids which may be employed in the preparation of the polyglycidyl esters of the present invention are polycarboxylic acids of general formula

$$(HO-\overset{\overset{O}{\|}}{C}-A)_m B-A-\overset{\overset{O}{\|}}{C}-OH \qquad II$$

wherein A, B and m have the same meaning as hereinbefore. Examples of polycarboxylic acids of general formula II include thiodipivalic acid, sulfinyldipivalic acid, sulfonyldipivalic acid, ethane-1,2-bis(thiopivalic acid), ethane-1,2-bis(sulfinylpivalic acid), ethane-1,2-bis (sulfonylpivalic acid), propane-1,2,3-tris(thiopivalic acid), propane-1,2,3-tris(sulfinylpivalic acid) and propane-1,2,3-tris(sulfonylpivalic acid), butane-1,4-bis-(thiopivalic acid), butane-1,4-bis(sulfinylpivalic acid) and butane-1,4-bis(sulfonylpivalic acid). Thiodipivalic acid is a preferred polycarboxylic acid of general formula II.

Polycarboxylic acids of general formula II, wherein B is $-SO_p-(CH_2)_4-SO_p-$ or $(SO_p-CH_2)_2CH-SO_p-$, are novel compounds.

The polycarboxylic acids of general formula II wherein B is $-SO_p-Q-SO_p-$ or $(SO_p-CH_2)_2CH-SO_p-$, and p is O can conveniently be prepared e.g. by the reaction of chloropivalic acid with the appropriate alkanepolythiol having from 2-10 carbon atoms as described by A.C. Bellaart et al, Z. anorg.allg. Chem. 412, 155-160 (1975).

A preferred and novel method for the preparation of said polycarboxylic acids is via the reaction of the dialkali metal or diammonium salt of mercaptopivalic acid with the appropriate dihalogen alkane having from 2-10 carbon atoms, followed by acidification of the reaction product.

The preparation of thiodipivalic acid (p is O and B is $-SO_p-$)can conveniently be conducted by stirring an aqueous emulsion of sodium chloropivalate for 24 hours at 20-23 °C with an aqueous solution of sodium sulfide and subsequently acidifying the reaction medium to provide the thiodipivalate as a precipitate; this method is known. A preferred and novel method for the preparation of thiodipivalic acid is via the reaction of the dialkali metal or diammonium salt of mercaptopivalic acid with pivalolactone at a temperature in the range of from 0-25 °C and preferably in the range of from 5-15 °C followed by acidification of the reaction product.

The polycarboxylic acids of general formula II, wherein p is 1 or 2, may be prepared from the corresponding acids wherein p is O by reacting a dialkyl ester of such an acid e.g. diethyl thiodipivalate, as a solution in a blend of glacial acetic acid and acetic anhydride with the desired amount of hydrogen peroxide (30 %m), which is added dropwise to said solution over a period of 3 hours. After removal of excess solvent the desired product may be isolated via acidification and recrystallization. This method is known. With the polyglycidyl esters of general formula I wherein p is 1 or 2 there is more than one method for their preparation, e.g.:

a) glycidation of polycarboxylic acids of general formula II wherein p has the value of 1 or 2, and

b) glycidation of polycarboxylic acids of general formula II wherein p is O, followed by oxidation of the thio group(s)-containing polyglycidyl esters thus obtained, to the corresponding sulfinyl or sulfonyl group(s) -containing compounds by reacting a solution of e.g. diglycidyl thiodipivalate in dichloromethane and glacial acetic acid at -10 °C with stepwise-added benzyltriethyl ammonium permanganate for one hour. After dilution with diethyl ether the reaction is stirred for 30 minutes, whereupon the mixture is filtered. After parifying the organic solution, the desired compound can be obtained via evaporation of the organic solvent.

Example I

Preparation of thiodipivalic acid

To a stirred solution of 26 mol of the disodium salt of mercaptopivalic acid in 10.1 l of water and having a temperature of 10 °C, 2600 g (26 mol) of pivalolactone was added over 4 hours and at such a rate that the temperature of the reactor contents was maintained at approximately 10 °C. As the reactor contents tended to solidify a further 1,5 l water was added during the pivalolactone addition. Upon completion of the lactone addition the solution was stirred for a further 2 h. The reactor contents were acidified with concentrated hydrochloric acid (6050 ml) to arrive at a pH 3. The thick white precipitate formed, was separated by filtration. The solid mass was stirred in 8 l water and refiltered. The crude damp product at this stage weighed 7308 g. A small portion (34.39 g) was dried to give a white powder (27.75 g) which had a m.p. 155-157 °C (lit. 163-164 °C) i.e. a yield of crude thiodipivalic acid (TDPA) of 5465 g, (90%). The crude acid was recrystallized from an industrial methylated spirit (IMS)/water mixture (4.5/10 1) at reflux. The solution was allowed to cool for two days, and the recrystallized TDPA was separated by filtration and washed with water/IMS 100:45 v/v. After drying at 90 °C / 0,5 mm, 4401 g of pure TDPA was obtained (72.3 % yield on lactone) having a m.p. 163-164 °C.

| | | | |
|---|---|---|---|
| Analysis found: | C, 50.7; | H, 7.8; | S, 13.8% |
| Calculated for: $C_{10}H_{18}O_4S$: | C, 51.2; | H, 7.8; | S, 13.8%. |

Acid value: 474 mg KOH/g.

Example II

Preparation of butane-1,4-bis(thiopivalic acid).

An aqueous solution of the disodium salt of mercaptopivalic acid (MPA) was prepared under nitrogen from MPA (2 mol, 268.4 g), sodium hydroxide (4 mol, 160 g) and water (800 ml). The solution was stirred at 70 °C and 1,4-dichlorobutane (1 mol, 91.5 g) was added over 20 min, during which time the temperature of the turbid reaction mixture remained at 70-80 °C. The reaction mixture was stirred until all the dichlorobutane had reacted (approx. 90 min), as a result of which the turbidity disappeared and the temperature had increased to 100 °C. The solution was stirred for a further 15 minutes at 100 °C, cooled, and acidified dropwise with concentrated hydrochloric acid (180 ml). The precipitated solid was filtered off, sucked-dry and recrystallized from diethyl ether / 30-40 °C boiling range petroleum spirit to give the pure acid, as a white solid, in 86.5% yield, M.p. 95.5-96 °C, equivalent weight 162.1.

| | | | |
|---|---|---|---|
| Analysis found: | C, 51.8; | H, 8.1; | S, 20.2%. |
| Calculated for $C_{14}H_{26}O_4S_2$: | C, 52.1; | H, 8.1; | S, 19.9%. |

Example III

Preparation of propane-1,2,3-tris(thiopivalic acid).

Propane-1,2,3-tris(thiopivalic acid) was prepared following the method as described in example II and employing 1 mol of 1,2,3-trichloropropane, 3 mol MPA and 6 mol NaOH. After recrystallization the acid was obtained in a 83 % yield. M.p: 118-121 °C, equivalent weight 148.3.

| | | | |
|---|---|---|---|
| Analysis found: | C, 48.8; | H, 7.3; | S, 22.9%. |
| Calculated for $C_{18}H_{32}O_6S_3$: | C, 49.1; | H, 7.3; | S, 21.8%. |

Example IV

Preparation of sulfonyldipivalic acid (SDPA)

Tungsten oxide (10 g, 0.04 mol) was stirred as a suspension in water (5 l) at 50 °C. A concentrated aqueous solution of sodium hydroxide was added dropwise to give an alkaline solution when all the oxide had dissolved. The solution was acidified to a pH 5-6 with glacial acetic acid to liberate tungstic acid, which compound is used as a catalyst in the preparation of the SDPA.

Thiodipivalic acid (936.8 g, 4.0 mol) was added to the tungstic acid containing solution and the resulting slurry was stirred at 60 °C. Next hydrogen peroxide (1000 ml of 30% m) was added dropwise to the slurry. During the addition of the first 500 ml (over 100 min) the temperature remained at 72-73 °C without external heating. During the addition of the remainder of the hydrogen peroxide (over 40 min) external heating was required to keep the temperature in the range of from 75-85 °C. Subsequently the slurry was stirred at 85-89 °C for 24 hours and then cooled. Crude SDPA was separated by filtration and washed free of peroxide with water. The crude SDPA was obtained in 90 % yield, m.p. 237-240 °C. After recrystallization from glacial acetic acid (5 l), washing with petroleum spirit (40-60 °C boiling range) and drying at 100 °C/5 mm Hg, there was obtained 866 g (81,5 %) of pure SDPA; mp 239.5-240.5 °C.

| | | | |
|---|---|---|---|
| Analysis Found: | C, 44.6; | H, 6.8; | S, 12.2% |
| Calculated for $C_{10}H_{18}O_6S$: | C, 45.1; | H, 6.8; | S, 12.1% |

Acid value 426 mg KOH/g.

Example V

Preparation of diglycidyl thiodipivalate

The disodium salt of thiodipivalic acid was prepared by adding 35,1 g (0.15 mol) thiodipivalic acid to a solution of 11,79 g sodium hydroxide in 58.8 ml water, and stirring this mixture for about 15 minutes. This was followed by removing the water by means of evaporation at reduced pressure and further drying at 50-75 °C and 100 mm Hg.

The disodium thiodipivalate was added to a stirred mixture of 215.0 g (2,32 mol) epichlorohydrin (ECH) and 0.91 g (2.82 x $10^{-3}$ mol) tetrabutyl ammonium bromide. Subsequently the mixture was refluxed with stirring, for 4 hours (on an oil bath 120 °C) and cooled to 40 °C and filtered. The excess ECH was removed by evaporation at a temperature of up to 120 °C and a pressure of mm Hg. The residue was added to 147 ml toluene, which was followed by the addition of 1.88 g sodium hydroxide in 27,5 ml water. The resulting two layer system was heated for 15 min. on an oil bath at 100 °C. The organic layer was separated off and washed with 300 ml of an aqueous 3 % m $NaH_2PO_4$ solution, which was followed by washing with water, drying over $MgSO_4$ and filtering. The solvent was evaporated under reduced pressure (15 mm Hg).

Vacuum distillation of the residue resulted in 39.4 g (yield 76 %) of a pale-yellow oil having a b.p. 167 °C/0.03 mm Hg and a viscosity of 91 mPa.s. $^1H$ NMR and 13C-NMR indicated the product to be diglycidyl thiodipivalate. The epoxy content was found to be 96.1 % of the theoretical amount and which epoxy content did not change during storage at 40 °C of 40 days.

Example VI

Preparation of diglycidyl sulfonyldipivalate

346 mg diglycidyl thiodipivalate (1 mmol) was dissolved in 10 ml dichloromethane which was followed by the addition of 1 g glacial acetic acid. The stirred solution was cooled to -10 °C with the aid of an ice/salt bath. Subsequently 684 mg benzyltriethyl ammonium permanganate was added stepwise i.e. 7

additions of approximately 98 mg, at a rate of 1 addition per min. After stirring the mixture for one hour the ice/salt bath was removed and 20 ml diethyl ether was added and after stirring a further 30 min. the mixture was filtered. The organic solution was shaken twice with 15 ml water and once with 15 ml of an aqueous 5 % m $NaHCO_3$ solution, dried with $MgSO_4$ and filtered. Finally the solvent was removed by evaporation in a film evaporator resulting in 240 mg of diglycidyl sulfonyldipivalate. The presence of the sulphonyl groups being confirmed by Infra Red Spectroscopy.

Example VII

Preparation of crosslinked diglycidyl esters of thiodipivalic acid

The diglycidyl ester of thiodipivalic acid as prepared in Example V was mixed with isophorone diamine (IPD) and diphenyldiamino methane (DDM) in amounts and under conditions as indicated in Table 1 hereinafter. The mixtures were transferred to an aluminium cup (Ø 5 cm) which had been pretreated with a silicon-based release agent (ex Dow Corning). The contents of the cups were heated for 1 h at 100 °C, followed by 1 h at 125 °C and finally for 4 h at 175 °C. The diglycidyl ester /IPD mixture was also applied as a 25 µm film on a phosphatized steel strip with the aid of a film applicator and subsequently heated in a nitrogen atmosphere for 90 min at 175 °C.

After curing all castings were hard, clear light coloured systems of which the Tg values have been included in Table 1. The IPD base film furthermore demonstrated very good adhesion properties to the metal substrate and a high gloss.

Table I

| Application | Casting | Casting | Film |
|---|---|---|---|
| Diglycidyl ester g | 2.415 | 2.333 | 3.14 |
| IPD g | 0.585 | - | 0.76 |
| DDM g | - | 0.667 | - |
| Mixing temp. °C | 20 | 100* | 20 |
| Tg after curing °C | 77 | 79 | - |

* The individual compounds were heated separately to 100 °C before mixing.

**Claims**

**1.** Polyglycidyl esters of general formula

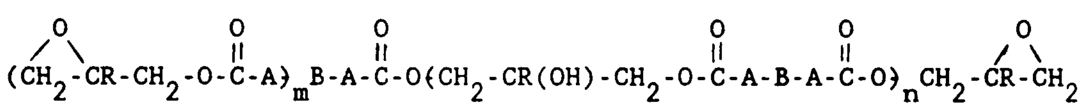

wherein R is H or $CH_3$; A represents an alkylene group $-CR^1_2-CH_2-$ wherein each $R^1$ represents a $C_1$-$C_4$ alkyl group which may be the same or different, which group A is attached to the adjacent group B via the $-CH_2-$ group; n has a value in the range of from 0-10; m is 1 or 2; for m is 1 B represents a group $-SO_p-$ or a group $-SO_p-Q-SO_p-$ wherein p is 0, 1 or 2, and Q is a linear or branched alkylene group having from 2-10 carbon atoms, for m is 2 B represents a group $(SO_p-CH_2)_2-CH-SO_p-$, wherein p has the same meaning as hereinbefore.

**2.** Polyglycidyl esters as claimed in claim 1, wherein B is $-SO_p-Q-SO_p-$ and Q is an ethylene group.

**3.** Polyglycidyl esters as claimed in claim 1, wherein B is $-SO_p-$.

**4.** Polyglycidyl esters as claimed in any one of claims 1-3, wherein each $R^1$ is $CH_3$.

5. A process for the preparation of polyglycidyl esters as claimed in any one of claims 1-4, wherein a polycarboxylic acid of general formula

$$(HO-\overset{O}{\overset{\|}{C}}-A)_m B-A-\overset{O}{\overset{\|}{C}}-OH$$

wherein A, B and m have the same meaning as hereinbefore is reacted with an excess of an epihalohydrin or a $\beta$-methylepihalohydrin by known methods.

6. A process as claimed in claim 5, wherein the polycarboxylic acid is thiodipivalic acid and the epihalohydrin is epichlorohydrin.

7. Polycarboxylic acids of general formula

$$(HO-\overset{O}{\overset{\|}{C}}-A)_m B-A-\overset{O}{\overset{\|}{C}}-OH$$

wherein A, B and m are as defined in Claim 1.

8. Butane-1,4-bis(thiopivalic acid), propane-1,2,3-tris(thiopivalic acid) as claimed in Claim 7.

9. Diglycidyl thiopivalate and diglycidyl sulfonyldipivalate. as claimed in Claim 1.

**Patentansprüche**

1. Polyglycidylester der allgemeinen Formel

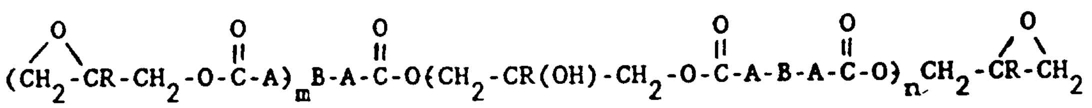

(I)

in der R Wasserstoff oder $CH_3$ ist; A eine Alkylengruppe - $CR^1{}_2$-$CH_2$- darstellt, wobei die Reste $R^1$ $C_1$-$C_4$-Alkylgruppen darstellen, die gleich oder verschieden sein können, wobei die Gruppe A über eine -$CH_2$- = Gruppe an die benachbarte Gruppe B gebunden ist; n einen Wert im Bereich von 0-10 hat; m 1 oder 2 ist; soweit m = 1 ist, stellt B eine Gruppe -$SO_p$- oder eine Gruppe -$SO_p$-Q-$SO_p$-dar, wobei p 0, 1 oder 2 ist und Q eine lineare oder verzweigte Alkylengruppe mit 2-10 Kohlenstoffatomen ist, soweit m = 2 ist, stellt B eine Gruppe -($SO_p$-$CH_2$)$_2$-CH-$SO_p$-dar, wobei p die gleiche Bedeutung wie vorstehend hat.

2. Polygylcidylester wie in Anspruch 1 beansprucht, wobei B -$SO_p$-Q-$SO_p$- ist und Q eine Ethylengruppe ist.

3. Polygylcidylester wie in Anspruch 1 beansprucht, wobei B -$SO_p$- ist.

4. Polyglycidylester wie in irgendeinem der Ansprüche 1-3 beansprucht, wobei jeder Rest $R^1$ $CH_3$ ist.

5. Ein Verfahren zur Herstellung von Polyglycidylestern wie in irgendeinem der Ansprüche 1-4 beansprucht, wobei eine Polycarbonsäure der allgemeinen Formel

$$(HO-\overset{O}{\overset{\|}{C}}-A)_m B-A-\overset{O}{\overset{\|}{C}}-OH \qquad (II)$$

in der A, B und m die gleiche Bedeutung wie vorstehend haben, mit einem Überschuß eines Ephihalogenhydrins oder eines $\beta$-Methylepihalogenhydrins mittels bekannter Methoden umgesetzt wird.

**6.** Ein Verfahren wie in Anspruch 5 beansprucht, wobei die Polycarbonsäure Thiodipivalinsäure ist und das Epihalogenhydrin Epichlorhydrin ist.

**7.** Polycarbonsäuren der allgemeinen Formel

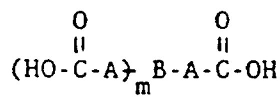

in der A, B und m wie in Anspruch 1 definiert sind.

**8.** Butan-1,4-bis(thiopivalinsäure), Propan-1,2,3-tris(thiopivalinsäure),wie in Anspruch 7 beansprucht.

**9.** Diglycidyl-thiopivalat und Diglycidyl-sulfonyldipivalat, wie in Anspruch 1 beansprucht.

## Revendications

**1.** Esters polyglycidiques de formule générale

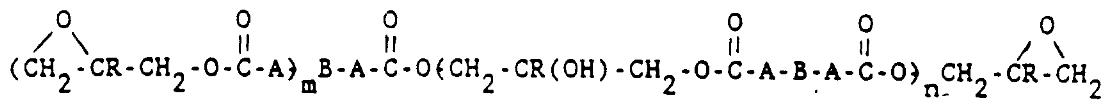

où R est H ou $CH_3$ ; A représente un groupe alcoylène $CR^1_2$ -$CH_2$- dans lequel chaque $R^1$ représente un groupe alcoyle en $C_1$-$C_4$, les deux pouvant être identiques ou différents, ce groupe A étant attaché au groupe B adjacent par l'intermédiaire du groupe -$CH_2$- ; n a une valeur comprise entre 0 et 10 ; m est 1 ou 2 ; quand m est 1, B représente un groupe -$SO_p$- ou un groupe -$SO_p$-Q-$SO_p$- où p est 0, 1 ou 2 et Q est un groupe alcoylène linéaire ou ramifié ayant de 2 à 10 atomes de carbone ; quand m est 2, B représente un groupe $(SO_p$-$CH_2)_2$-CH-$SO_p$-, où p a la même signification que ci-dessus.

**2.** Esters polyglycidiques selon la revendication 1, dans lesquels B est -$SO_p$-Q-$SO_p$- et Q est un groupe éthylène.

**3.** Esters polyglycidiques selon la revendication 1, dans lesquels B est -$SO_p$-.

**4.** Esters polyglycidiques selon l'une quelconque des revendications 1-3, dans lesquels chaque $R^1$ est $CH_3$.

**5.** Un procédé pour la préparation d'esters polyglycidiques selon l'une quelconque des revendications 1-4, dans lequel un acide polycarboxylique de formule générale

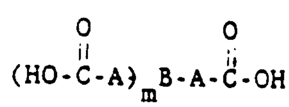

où A, B et m ont la même signification que ci-dessus est mis à réagir avec une épihalohydrine ou une bêta-méthylépihalohydrine par des méthodes connues.

**6.** Un procédé selon la revendication 5, dans lequel l'acide polycarboxylique est l'acide thiodipivalique et l'épihalohydrine est l'épichlorhydrine.

7. Acides polycarboxyliques de formule générale

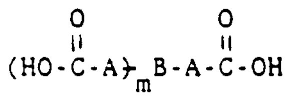

où A, B et m sont tels que définis dans la revendication 1.

8. Butane-1,4-bis(acide thiopivalique) et propane-1,2,3-tris(acide thiopivalique) selon la revendication 7.

9. Thiopivalate de diglycidyle et sulfonyldipivalate de diglycidyle selon la revendication 1.